# FASCICULE DE BREVET EUROPEEN

(11) **EP 0 950 387 B1**
(45) Date de publication et mention de la délivrance du brevet: **17.03.2004**
(21) Numéro de dépôt: 99400900.9
(22) Date de dépôt: 13.04.1999
(51) Int. Cl.: A61F 2/38

(54) **Prothèse de genou**
Knieprothese
Knee prosthesis

(30) Priorité: 15.04.1998 FR 9804665
(43) Date de publication de la demande: 20.10.1999
(73) Titulaire: Aesculap AG & Co. KG, 78532 Tuttlingen (DE)
(72) Inventeur: Marceaux, Pascal, 52000 Chaumont (FR); Biegun, Jean-François, 52000 Chaumont (FR); Jenny, Jean-Yves, 67640 Lipsheim (FR); Barba, Laurent, 69130 Ecully (FR); Hummer, Jacques, 54000 Nancy (FR); Catonne, Yves, 97280 le Vauclin (FR); Barthelemy, Jean-Paul, 37230 Fondettes (FR); Miehlke, Rolf, 48167 Münster (DE); Saragaglia, Dominique, 38640 Claix (FR)
(74) Mandataire: Eidelsberg, Victor Albert

(56) Documents cités:
- EP-A- 0 194 326
- WO-A-95/17860
- WO-A-98/02116
- FR-A- 2 676 916
- GB-A- 2 312 166
- US-A- 5 019 103

## Description

La présente invention est relative aux prothèses de genou.

D'une manière générale, ces prothèses comportent un élément prothétique fémoral destiné à être fixé à l'extrémité distale, ou inférieure, du fémur, un élément prothétique tibial destiné à être fixé à l'extrémité proximale, ou supérieure, du tibia, et au moins un élément prothétique méniscal intermédiaire qui présente au moins une surface supérieure concave de coopération par glissement avec une surface condylienne inférieure convexe de l'élément fémoral.

Parmi ces prothèses, l'invention concerne celles qui sont du type à élément méniscal mobile, par contraste avec les prothèses dans lesquelles cet élément méniscal est fixe par rapport à l'élément tibial. L'élément meniscal mobile peut se déplacer par rapport à l'élément tibial suivant un mouvement général antéro-postérieur de translation, en étant guidé par exemple par un rail, et éventuellement suivant un mouvement additionnel de rotation d'amplitude limitée autour d'un axe, lequel est perpendiculaire à la direction de la translation.

Parmi ces prothèses à élément méniscal mobile, l'invention s'applique aussi bien aux prothèses dites unicompartimentales, c'est-à-dire aux prothèses destinées à remplacer l'articulation d'un seul condyle, qu'aux prothèses dites tricompartimentales, c'est-à-dire aux prothèses destinées à remplacer les articulations des deux condyles, avec conservation du ligament croisé postérieur.

L'invention a pour but de fournir une prothèse de ce type qui restitue la pente antéro-postérieure anatomique de la face supérieure de l'embase tibiale, tout en assurant une préservation maximale du capital osseux et en simplifiant la coupe ou résection tibiale, laquelle se fait généralement dans un plan perpendiculaire à l'axe anatomique du tibia.

A cet effet, la prothèse de genou, selon l'invention, est telle que définie à la revendication 1. Des perfectionnements font l'objet des revendications 2 à 14. Ainsi, la prothèse, selon l'invention, restitue non seulement la mobilité anatomique du ou des ménisques, mais également la pente anatomique antéro-postérieure du genou, tout en préservant au maximum le capital osseux et en permettant un réglage simple du plan de résection tibiale, par exemple à 90° par rapport à l'axe anatomique ou mécanique du tibia. Le document GB-A-2312166 (D1) décrit une prothèse de genou suivant le préambule de la revendication 1. Dans ce document, l'axe perpendiculaire au plan défini par la surface supérieure 24 de l'embase et la surface inférieure de l'élément méniscal est indiné par rapport à l'axe de la quille. Ainsi, dans ce cas, pour restituer la pente AP du genou il est nécessaire d'effectuer une résection inclinée par rapport à l'axe anatomique du tibia. Le document US-A-5019103(D2) décrit une prothèse de genou dans laquelle la surface inférieure de l'embase n'est pas perpendiculaire à l'axe de la quille et l'axe perpendiculaire au plan défini par la surface supérieure de l'embase et la surface inférieure de l'élément méniscal n'est pas inclinée par rapport à l'axe de la quille. Dans ce cas également, il n'est pas possible d'effectuer une résection tibiale perpendiculairement à l'axe anatomique du tibia si l'on souhaite restituer la pente AP genou. Le document 2676916 (D3) décrit une prothèse dans laquelle l'axe perpendiculaire au plan défini par la surface supérieure de l'embase et la surface inférieure de l'élément méniscal n'est pas inclinée par rapport à l'axe de la quille. Dans ce cas, on ne peut pas restituer la pente AP du genou, à moins d'effectuer une coupe qui est inclinée par rapport à l'axe anatomique du tibia ce qui n'est pas souhaité.

Suivant un second mode de réalisation, ces surfaces planes présentent également une pente dans la direction médio-latérale, lesdites surfaces étant inclinées vers le bas et vers le plan sagittal, ou antéro-postérieur, du tibia. Dans ce cas, pour une prothèse du type tricompartimental, l'élément méniscal est en deux parties distinctes susceptibles de se déplacer chacune, par rapport à l'élément tibial, à la fois dans la direction antéro-postérieure et dans la direction médio-latérale, alors que dans le premier mode de réalisation, l'élément méniscal peut être en une seule partie ou en deux parties distinctes.

Dans le cas d'une prothèse du type unicompartimental, l'élément méniscal est bien évidemment en une seule partie, puisqu'il remplace un seul ménisque naturel.

Dans les deux modes de réalisation ci-dessus, pour une prothèse du type tricompartimental, l'élément méniscal, qu'il soit constitué d'une seule partie ou de deux parties distinctes, comporte deux ménisques prothétiques externe et interne coopérant respectivement avec deux condyles prothétiques externe et interne de l'élément fémoral.

Suivant une caractéristique avantageuse de l'invention, dans le cas d'une prothèse du type tricompartimental, le bord postérieur de la surface supérieure concave du ménisque prothétique externe est plus haut que le bord postérieur de la surface supérieure concave du ménisque prothétique interne, par rapport à un plan perpendiculaire à l'axe du tibia. Cette différence de hauteur des bords postérieurs des deux ménisques prothétiques a une valeur comprise entre 1 mm et 4 mm, de préférence 2 mm.

Suivant une autre caractéristique avantageuse de l'invention, également dans le cas d'une prothèse du type tricompartimental, le bord postérieur de la surface supérieure concave du ménisque prothétique externe est situé plus en arrière que le bord postérieur de la surface supérieure concave du ménisque prothétique interne, par rapport au plan médio-latéral du tibia. Cet écart de position des bords postérieurs des deux ménisques prothétiques a une valeur comprise entre 1 mm et 4 mm, de préférence 2 mm.

Par exemple, la surface méniscale supérieure concave de l'élément méniscal et la surface condylienne inférieure convexe de l'élément fémoral sont des surfaces toriques congruentes à génératrice en arc de cercle.

on comprendra bien l'invention à la lecture du complément de description qui va suivre et en référence aux dessins annexés qui font partie de la description et dans lesquels :
Fig. 1 est une vue en élévation antérieure d'une prothèse de genou tricompartimentale établie suivant un mode de réalisation préféré de l'invention ;
Fig. 2 est une vue en plan de dessus de la prothèse de la Fig. 1 sans l'élément prothétique fémoral ;
Fig. 3 est une vue latérale de gauche de la prothèse de la Fig. 1 ;
Fig. 4 est une coupe suivant la ligne IV-IV de la Fig. 3 ;
Fig. 5 est une vue en élévation postérieure de la prothèse de la Fig. 1 ;
Fig. 6 est une coupe suivant la ligne VI-VI de la Fig. 5 ; et
Fig. 7 est une coupe suivant la ligne VII-VII de la Fig. 5.

On a représenté sur les dessins une prothèse de genou tricompartimentale, qui, d'une manière générale, comporte un élément prothétique fémoral 1 destiné à être fixé à l'extrémité distale, ou inférieure, du fémur, un élément prothétique tibial 2 destiné à être fixé à l'extrémité proximale, ou supérieure, du tibia, et un élément prothétique méniscal intermédiaire mobile 3 destiné à coopérer avec l'élément fémoral 1 et l'élément tibial 2.

L'élément fémoral 1, de manière connue, est enveloppant, et il est ancré dans le fémur par deux plots d'ancrage 4. Par ailleurs, son logement 5 de réception du fémur coopère étroitement avec des surfaces de résection ménagées à l'extrémité distale du fémur, à savoir une surface antérieure 6, une surface postérieure 7, une surface distale 8, et deux surfaces de chanfreins 9 et 10. La surface distale 8 est perpendiculaire à l'axe anatomique ou mécanique 11 du fémur.

L'élément fémoral 1 est en une seule pièce et il comporte une partie trochléenne antérieure 12 qui se raccorde à sa partie inférieure à deux condyles 13 et 14.

L'élément tibial 2 comporte une quille nervurée 15 d'ancrage dans le tibia et une embase 16 dont la surface inférieure 17 est plane et est destinée à venir en appui contre un plan de résection proximale ménagé sur le tibia, perpendiculairement à l'axe anatomique ou mécanique 18 de celui-ci.

L'élément méniscal 3 est en forme de plateau et il est mobile à la fois par rapport à l'élément fémoral 1 et par rapport à l'élément tibial 2.

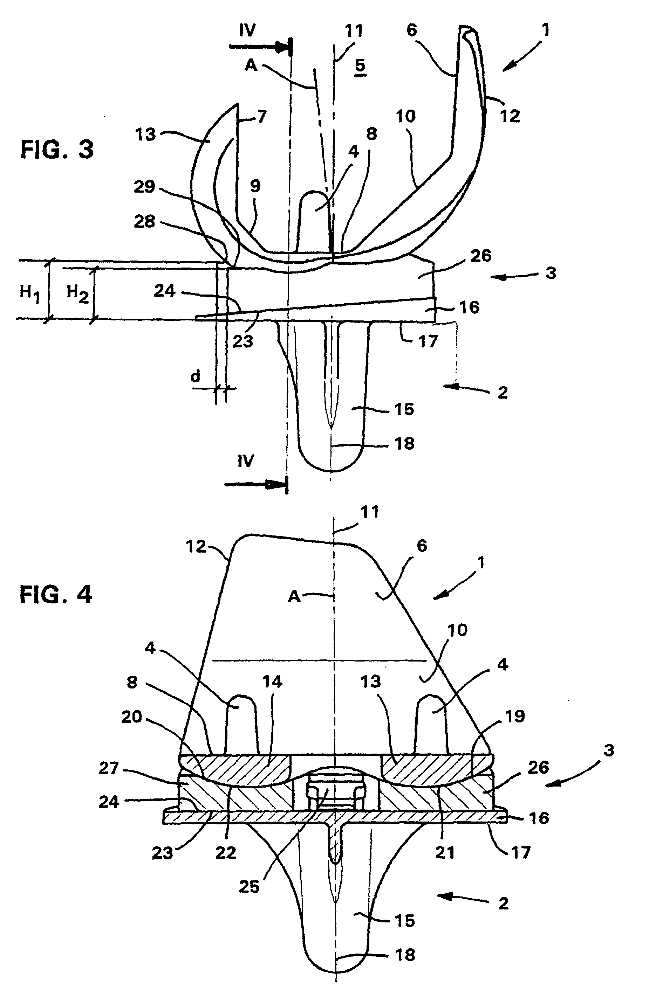

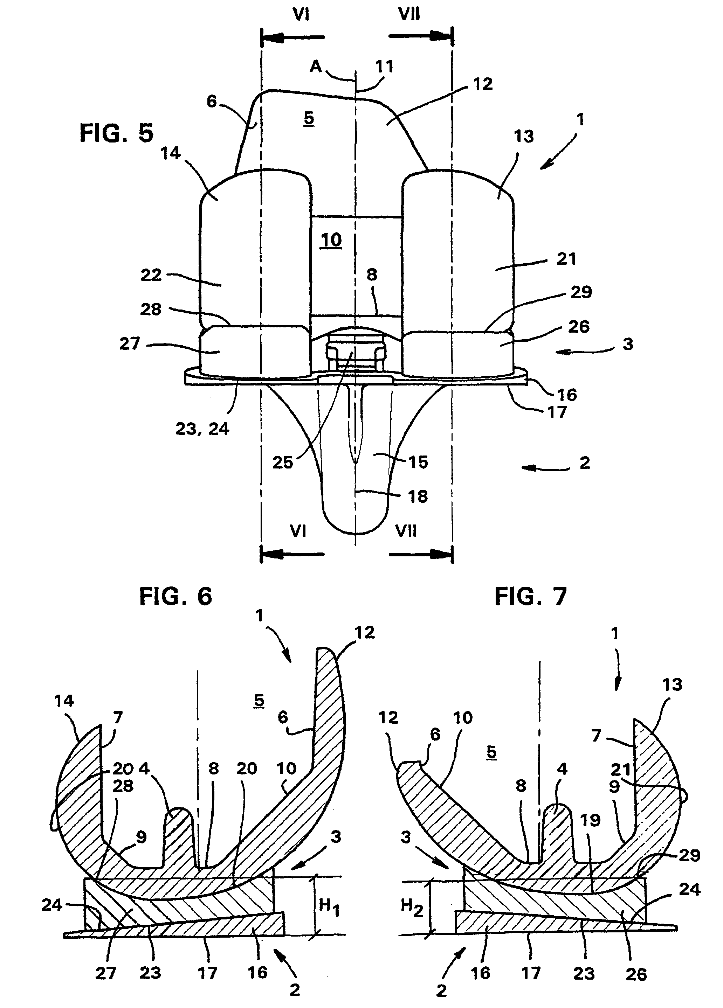

Pour sa coopération avec chacun des condyles 13 et 14, l'élément méniscal 3 présente une surface supérieure concave 19, 20 en forme de tore à génératrice en arc de cercle, comme cela apparaît notamment sur les Figs. 4, 6 et 7. L'axe des surfaces toriques 19 et 20 est parallèle à la direction médio-latérale.

Pour coopérer avec les surfaces toriques 19 et 20, les condyles 13 et 14 présentent des surfaces inférieures convexes respectives 21 et 22 qui sont en congruence avec les surfaces concaves 19 et 20, c'est-à-dire qu'elles sont également toriques.

Le mouvement relatif de l'élément fémoral 1 et de l'élément méniscal 3 est donc un mouvement de roulement avec glissement.

L'élément méniscal 3 coopère par glissement de translation avec l'élément tibial 2 par des surfaces planes de ces deux éléments qui sont inclinées du même angle vers le haut de l'arrière vers l'avant, comme on le voit au mieux sur les Figs. 3, 6 et 7.

Pour cette coopération, l'élément méniscal 3 présente une surface inférieure plane 23, et l'élément tibial 2 présente une surface supérieure plane 24. Dans le mode de réalisation représenté, les surfaces planes 23 et 24 sont inclinées seulement dans la direction antéro-postérieure du tibia, c'est-à-dire sans pente dans la direction médio-latérale.

L'angle d'inclinaison de ces surfaces planes, par rapport à un plan perpendiculaire à l'axe 18 du tibia, c'est-à-dire par rapport au plan de la surface inférieure 17 de l'embase 16, présente avantageusement une valeur comprise entre 1° et 10°, de préférence 5°.

Suivant une variante, non représentée, les surfaces 23 et 24 pourraient également présenter une pente dans la direction médio-latérale, en étant inclinées vers le bas et vers le plan sagittal, ou antéro-postérieur, du tibia. Dans ce cas, pour une prothèse tricompartimentale, chaque surface 24 est constituée par deux facettes planes en forme de V, et l'élément méniscal 3 est en deux parties distinctes qui sont susceptibles chacune de se déplacer, par rapport à l'élément tibial 2, à la fois dans la direction antéro-postérieure et dans la direction médio-latérale.

Dans l'exemple de prothèse tricompartimentale représenté, dans lequel il n'existe une pente que dans la direction antéro-postérieure, l'élément méniscal 3 est de préférence en une seule partie.

De manière connue en soi, l'élément méniscal 3 peut être guidé, dans son mouvement général de translation antéro-postérieure par rapport à l'élément tibial 2, par tous moyens appropriés schématisés en 25, par exemple un rail, porté par l'embase 16. Le cas échéant, le rail 25 peut tourner par rapport à l'embase 16 autour d'un axe A situé dans le plan sagittal. Dans le cas où il n'existe qu'une pente antéro-postérieure, cet axe est perpendiculaire aux surfaces planes 23, 24, pour que l'élément méniscal ait un mouvement composé de translation et de rotation par rapport à l'élément tibial 2. Dans le cas où il existe également une pente médio-latérale, cet axe A est perpendiculaire à la ligne d'intersection des deux facettes planes formant la surface supérieure 24 de l'élément tibial.

La pente antéro-postérieure qui est donnée aux surfaces de glissement coopérantes de l'élément méniscal 3 et de l'élément tibial 2 permet de s'opposer à toute luxation, c'est-à-dire à une tendance du ménisque prothétique à être éjecté vers l'avant, lors de la flexion, laquelle peut s'effectuer avec congruence totale entre 0° et environ 140°.

Un autre avantage de cette pente antéro-postérieure réside dans la suppression du mouvement dit de "tilting", c'est-à-dire le basculement du ménisque sous la pression du condyle associé par soulèvement du ménisque dans sa partie antérieure.

La prothèse tricompartimentale représentée sur les dessins est asymétrique et, en l'occurrence, il s'agit d'une prothèse du genou gauche. La prothèse correspondante de genou droit est symétrique de celle qui est représentée par rapport à un plan antéro-postérieur.

Dans la configuration représentée, l'élément méniscal 3 comporte donc un ménisque prothétique interne 26 et un ménisque prothétique externe 27, coopérant respectivement avec le condyle prothétique interne 13 et le condyle prothétique externe 14. Comme indiqué précédemment, les ménisques prothétiques 26 et 27 sont constitués par une partie unique ou par deux parties distinctes.

Suivant une caractéristique avantageuse de l'invention applicable à ce type de prothèse tricompartimentale, on limite les risques de luxation de la partie externe de la prothèse, dont on sait que la stabilité est moindre que du côté interne, en prévoyant pour le condyle externe 14 une surface de coopération avec le ménisque externe 27 qui est plus importante que la surface de coopération entre le condyle interne 13 et le ménisque interne 26.

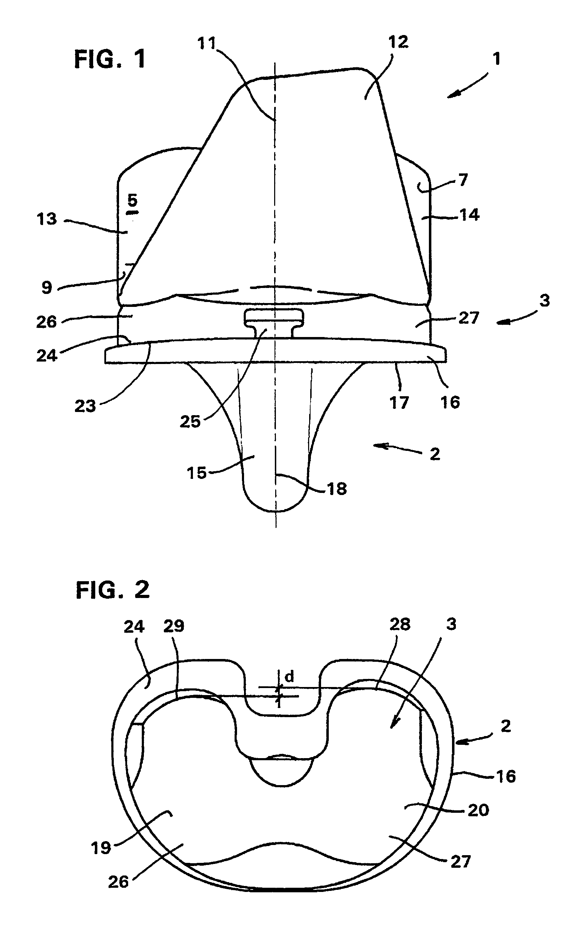

Cela se traduit, comme montré au mieux sur les Figs. 2 et 3, par le fait que le bord postérieur 28 de la surface supérieure concave 20 du ménisque externe 27 est situé plus en arrière que le bord postérieur 29 de la surface supérieure concave 19 du ménisque interne 26, par rapport au plan médio-latéral. Cet écart de position entre les bords postérieurs 28 et 29 est représenté en d sur les Figs. 2 et 3, et il présente une valeur comprise entre 1 mm et 4 mm, de préférence 2 mm.

Cet écart de position des bords postérieurs des surfaces supérieures concaves des ménisques 26 et 27 se traduit également, comme montré au mieux sur les Figs. 3, 5, 6 et 7, par le fait que le bord postérieur 28 de la surface 20 du ménisque externe 27 est plus haut que le bord postérieur 29 de la surface 19 du ménisque interne 26, par rapport à un plan perpendiculaire à l'axe du tibia, c'est-à-dire par rapport au plan de la surface inférieure 17 de l'embase 16 de l'élément tibial 2.

on voit sur les Figs. 3, 6 et 7 que, par rapport à ce plan de référence, la hauteur H₁ du bord postérieur 28 est supérieure à la hauteur H₂ du bord postérieur 29.

La différence entre ces deux hauteurs présente avantageusement une valeur qui est comprise entre 1 mm et 4 mm, de préférence 2 mm.

Par ailleurs, comme cela apparaît clairement sur les Figs. 3, 6 et 7, l'élément méniscal 3, du fait de l'inclinaison antéro-postérieure des surfaces de glissement 23, 24, se présente sous la forme d'un coin dont la partie antérieure est amincie par rapport à la partie postérieure.

Bien entendu, l'invention n'est pas limitée aux modes de réalisation qui ont été décrits ; on pourrait au contraire concevoir diverses variantes sans sortir pour autant de son cadre.

## Revendications

1. Prothèse de genou, comportant un élément prothétique fémoral (1) destiné à être fixé à l'extrémité distale, ou inférieure, du fémur, un élément prothétique tibial (2) comportant une embase (16) dont la surface inférieure (17) est plane et est destinée à venir en appui sur un plan de résection proximal du tibia, et au moins un élément prothétique méniscal intermédiaire (3) mobile au moins dans la direction antéro-postérieure et présentant une surface inférieure (23) plane de coopération par glissement avec une surface supérieure complémentaire (24) de l'embase, et au moins une surface supérieure concave (19, 20) de coopération par glissement avec une surface condylienne inférieure convexe (21, 22) de l'élément fémoral, la surface inférieure (17) de l'embase (16) étant plane et perpendiculaire à l'axe de la quille (15), **caractérisée en ce que** l'axe perpendiculaire au plan défini par la surface supérieure (24) de l'embase (16) et la surface inférieure (23) de l'élément méniscal est incliné, par rapport à l'axe de la quille.

2. Prothèse selon la revendication 1, **caractérisée en ce que** l'angle d'inclinaison desdites surfaces planes a une valeur comprise entre 1° et 10°.

3. Prothèse selon l'une des revendications 1 et 2, **caractérisée en ce que** lesdites surfaces planes (23, 24) sont inclinées seulement dans la direction antéro-postérieure, c'est-à-dire sans pente dans la direction médio-latérale.

4. Prothèse selon l'une des revendications 1 et 2, **caractérisée en ce que** lesdites surfaces planes présentent également une pente dans la direction médio-latérale, lesdites surfaces étant inclinées vers le bas et vers le plan sagittal, ou antéro-postérieur, du tibia.

5. Prothèse selon la revendication 4, **caractérisée en ce que** l'élément méniscal est en deux parties distinctes susceptibles de se déplacer chacune, par rapport à l'élément tibial, à la fois dans la direction antéro-postérieure et dans la direction médio-latérale.

6. Prothèse selon l'une des revendications 1 à 3, **caractérisée en ce que** l'élément méniscal est en une seule partie.

7. Prothèse selon l'une des revendications 1-4 et 6, **caractérisée en ce qu'**elle est du type unicompartimental.

8. Prothèse selon l'une des revendications 1 à 6, **caractérisée en ce qu'**elle est du type tricompartimental, l'élément méniscal comportant, en une seule partie ou en deux parties distinctes, deux ménisques prothétiques externe (27) et interne (26) coopérant respectivement avec deux condyles prothétiques externe (14) et interne (13) de l'élément fémoral.

9. Prothèse selon la revendication 8, **caractérisée en ce que** le bord postérieur (28) de la surface supérieure concave (20) du ménisque prothétique externe (27) est plus haut que le bord postérieur (29) de la surface supérieure concave (19) du ménisque prothétique interne (26), par rapport à un plan perpendiculaire à l'axe du tibia.

10. Prothèse selon la revendication 9, **caractérisée en ce que** la différence des hauteurs (H₁, H₂) desdits bords postérieurs (28, 29) présente une valeur comprise entre 1 mm et 4 mm, de préférence 2 mm.

11. Prothèse selon l'une des revendications 8 à 10, **caractérisée en ce que** le bord postérieur (28) de la surface supérieure concave (20) du ménisque prothétique externe (27) est situé plus en arrière que le bord postérieur (29) de la surface supérieure concave (19) du ménisque prothétique interne (26), par rapport au plan médio-latéral du tibia.

12. Prothèse selon la revendication 11, **caractérisée en ce que** l'écart (d) des positions desdits bords postérieurs (28, 29) présente une valeur comprise entre 1 mm et 4 mm, de préférence 2 mm.

13. Prothèse selon l'une des revendications 1 à 12, **caractérisée en ce que** l'élément méniscal est en forme de coin dont la partie antérieure est amincie par rapport à la partie postérieure.

14. Prothèse selon l'une des revendications 1 à 13, **caractérisée en ce que** la surface méniscale supérieure concave (19, 20) de l'élément méniscal et la surface condylienne inférieure convexe (21, 22) de l'élément fémoral sont des surfaces toriques congruentes à génératrice en arc de cercle.

## Claims

1. A knee prosthetic, incorporating a femoral prosthetic component (1) designed to be fixed to the distal or lower extremity of the femur, a tibial prosthetic component (2) incorporating an embase (16) of which the lower surface (17) is flat and is designed to lean on a close resection plane of the tibia, and at least one intermediary meniscoid prosthetic component (3), mobile at least in the ante-posterior direction and having a lower plane surface (23) configured to work together by sliding with one complementary upper surface (24) of the embase, and at least one upper concave surface (19, 20) configured to work together by sliding with one lower convex condyloid surface (21,22) of the femoral component, wherein the lower surface(17) of the embase (16) is flat and perpendicular to the axis of the leg (15) for anchorage, **characterised in that** the axis perpendicular to the plane defined by the top surface (24) of the embase (16) and the lower surface (23) of the meniscoid component is tilted with respect to the axis of the leg for anchorage.

2. A prosthetic according to claim 1, **characterised in that** the angle of tilt of the said flat surfaces is between one degree and ten degrees.

3. A prosthetic according to claim 1 or claim 2, **characterised in that** said flat surfaces (23, 24) are only sloping in the ante-posterior direction, with no gradient in the medio-lateral direction.

4. A prosthetic according to claim 1 or claim 2, **characterised in that** said flat surfaces also involve a gradient in the medio-lateral direction, the said surfaces being sloped towards the bottom and towards the sagittal or ante-posterior plane of the tibia.

5. A prosthetic according to claim 4, **characterised in that** the meniscoid component is in two different parts each able to move about, with respect to the tibial component, in the ante-posterior direction and in the medio-lateral direction at the same time.

6. A prosthetic according to any of claims 1 to 3, **characterised in that** the meniscoid component is in a single part.

7. A prosthetic according to any of claims 1 to 4 and 6, **characterised in that** said prosthetic is uni-compartmental.

8. A prosthetic according to any of claims 1 to 6, **characterised in that** said prosthetic is three-compartmental, the meniscoid component incorporating, in a single part or in two different parts, two prosthetic menisci external (27) and internal (26) working together with two prosthetic condyles external (14) and internal (13) respectively of the femoral component.

9. A prosthetic according to claim 8, **characterised in that** the rear edge (28) of the upper concave surface (20) of the external prosthetic meniscus (27) is higher than the rear edge (29) of the upper concave surface (19) of the internal prosthetic meniscus (26) with respect to a perpendicular plane to the axis of the tibia.

10. A prosthetic according to claim 9, **characterised in that** the difference in heights (H1, H2) of said rear edges (28, 29) involve a value between 1 mm and 4mm, preferably 2 mm.

11. A prosthetic according to any of claims 8 to 11, **characterised in that** the rear edge (28) of the upper concave surface (20) of the external prosthetic meniscus (27) is situated further back than the rear edge (29) of the upper concave surface (19) of the internal prosthetic meniscus (26), with respect to the medio-lateral plane of the tibia.

12. A prosthetic according to claim 12, **characterised in that** the distance (d) between said rear edges is between 1mm and 4mm, preferably 2mm.

13. A prosthetic according to any of claims 1 to 12, **characterised in that** the meniscoid component is in the form of a comer of which the anterior part is narrowed with respect to the rear part.

14. A prosthetic according to any of claims 1 to 13, wherein the upper concave meniscoid surface (19, 20) of the meniscoid component and the lower convex condyloid surface (21, 22) of the femoral component are toric surfaces able to generate an arc of a circle.

## Patentansprüche

1. Knieprothese mit einem femoralen Protheseelement (1), das dazu vorgesehen ist, an einem distalen oder unteren Ende des Femurs befestigt zu werden, einem tibialen Protheseelement (2) mit einer Basis (16), die an der unteren Oberfläche (17) plan ist und dazu vorgesehen ist, an einer proximalen Resektionsfläche der Tibia anzuliegen, und mindestens einem meniskalen Zwischen-Protheseelement (3), das mindestens in der Richtung anterior-posterior beweglich ist und eine untere plane Oberfläche (23) zum Zusammenwirken durch Verschiebung mit einer oberen komplementären Oberfläche (24) der Basis sowie mindestens eine konkave obere Oberfläche (19,20) zum Zusammenwirken durch Verschiebung mit einer unteren konvexen condylaren Oberfläche (21,22) des femoralen Elements aufweist, wobei die untere Oberfläche (17) der Basis (16) plan und senkrecht zur Achse des Kiels (15) ist,
**dadurch gekennzeichnet, dass** die Achse, die zu dem durch die obere Oberfläche (24) der Basis (16) und die untere Oberfläche (23) des meniskalen Elements definierten Ebene senkrecht ist, in Bezug auf die Kielachse geneigt ist.

2. Prothese nach Anspruch 1, **dadurch gekennzeichnet, dass** der Neigungswinkel der planen Oberflächen einen zwischen 1° und 10° liegenden Wert aufweist.

3. Prothese nach einem der Ansprüche 1 oder 2, **dadurch gekennzeichnet, dass** die planen Oberflächen (23,24) nur in der Richtung anterior-posterior geneigt sind, das heisst, ohne Neigung in der medio-lateralen Richtung sind.

4. Prothese nach einem der Ansprüche 1 oder 2, **dadurch gekennzeichnet, dass** die planen Oberflächen (23,24) auch eine Neigung in der medio-lateralen Richtung aufweisen, wobei die Oberflächen nach unten und zu der sagittalen Ebene, oder anterior-posterior, der Tibia geneigt sind.

5. Prothese nach Anspruch 4, **dadurch gekennzeichnet, dass** das meniskale Element aus zwei unterschiedlichen Abschnitten besteht, die sich jeweils in Bezug auf das tibiale Element sowohl in der anterior-posterioren Richtung als auch in der medio-lateralen Richtung bewegen können.

6. Prothese nach einem der Ansprüche 1 bis 3, **dadurch gekennzeichnet, dass** das meniskale Element aus einem einzigen Teil besteht.

7. Prothese nach einem der Ansprüche 1 bis 4 und 6, **dadurch gekennzeichnet, dass** es vom ungeteilten Typ ist.

8. Prothese nach einem der Ansprüche 1 bis 6, **dadurch gekennzeichnet, dass** es vom dreigeteiltenTyp ist, wobei das meniskale Element in einem einzigen Abschnitt oder in zwei unterschiedlichen Abschnitten zwei prothetische Menisken, einen externen (27) und einen internen (26), aufweist, die jeweils mit zwei prothetischen Condylen, einem externen (14) und einem internen (13), des femoralen Elements zusammenwirken.

9. Prothese nach Anspruch 8, **dadurch gekennzeichnet, dass** der hintere Rand (28) der oberen konkaven Oberfläche (20) des externen prothetischen Meniskus (27) in Bezug auf eine zur Achse der Tibia senkrechte Ebene höher ist als der hintere Rand (29) der oberen konkaven Oberfläche (19) des internen prothetischen Meniskus (26).

10. Prothese nach Anspruch 9, **dadurch gekennzeichnet, dass** der Unterschied der Höhen (H₁,H₂) der hinteren Ränder (28,29) einen zwischen 1 mm und 4 mm liegenden Wert, vorzugsweise 2 mm, aufweist.

11. Prothese nach einem der Ansprüche 8 bis 10, **dadurch gekennzeichnet, dass** der hintere Rand (28) der konkaven oberen Oberfläche (20) des externen prothetischen Meniskus (27) in Bezug auf die medio-laterale Ebene der Tibia weiter hinten gelegen ist als der hintere Rand (29) der konkaven oberen Oberfläche (19) des internen prothetischen Meniskus (26).

12. Prothese nach Anspruch 11, **dadurch gekennzeichnet, dass** der Abstand (d) der Positionen der hinteren Ränder (28,29) einen zwischen 1 mm und 4 mm liegenden Wert, vorzugsweise 2 mm, aufweist.

13. Prothese nach einem der Ansprüche 1 bis 12, **dadurch gekennzeichnet, dass** das meniskale Element die Form eines Keils aufweist, dessen Vorderabschnitt in Bezug auf den hinteren Abschnitt dünner ist.

14. Prothese nach einem der Ansprüche 1 bis 13, **dadurch gekennzeichnet, dass** die obere konkave meniskale Oberfläche (19,20) des meniskalen Elements und die untere konvexe Oberfläche des Condylus (21,22) des femoralen Elements kongruente torische Oberflächen mit einer Kreisbogen-Generatrix sind.
